# EUROPEAN PATENT SPECIFICATION

(11) **EP 0 889 993 B1**
(45) Date of publication and mention of the grant of the patent: **28.06.2000**
(21) Application number: 97915811.0
(22) Date of filing: 21.03.1997
(51) Int. Cl.: D21H 17/63, D21H 21/22

(54) **ABSORBENT CELLULOSIC MATERIAL AND PRODUCTION THEREOF**
ABSORBIERENDES ZELLSTOFFHALTIGES MATERIAL UND VERFAHREN ZUR HERSTELLUNG
MATERIAU ABSORBANT CELLULOSIQUE ET PRODUCTION DE CELUI-CI

(30) Priority: 25.03.1996 SE 9601135
(43) Date of publication of application: 13.01.1999
(73) Proprietor: Eka Chemicals AB, 445 80 Bohus (SE)
(72) Inventor: THEBRIN, Ingemar, S-444 41 Stenungsund (SE); WAHLEN, Svante, S-444 42 Stenungsund (SE); LINDGREN, Erik, S-445 34 Bohus (SE); MALMBORG, Kerstin, S-440 74 Hjälteby (SE)
(74) Representative: Jönsson, Christer
(86) International application number: SE9700506
(87) International publication number: WO9736046

(56) References cited:
- EP-A- 0 540 076
- WO-A-91/11977
- US-A- 4 826 497

## Description

The present invention relates to a method for production of fluff having improved sorptive capacity, and to fluff obtainable by such a method. The invention also relates to the use of hydrophobic substances having a specific surface area of more than 50 m²/g for treatment of cellulosic fibres to improve the sorptive capacity of absorbent material comprising the fibres. Furthermore, the invention relates to a process for production of an absorbent material comprising cellulosic fibres obtained by shredding or fluffing a pulp sheet, which structure has improved sorptive capacity.

In the present context the concept of "sorptive capacity" aims at the rate by which the absorbent material takes up liquids such as water or aqueous solutions, including body fluids such as urine, blood and menstrual fluids, or to the liquid-retaining capacity of the absorbent material, or to both of these characteristics. The sorptive mechanism may be adsorption or absorption, or a combination thereof.

As stated in Pulp and Paper Manufacture, Vol. 2, page 280-281, Joint Textbook Committee of the Paper Industry, 1987, the term fluff applies to fibres which have been separated by mechanical means from dry pulps for application in dry-formed nonwoven webs or pads in the household or sanitary fields. As is evident from this reference both the rate of liquid uptake and liquid-holding capacity of fluff are important when used in these fields. This is particularly valid with regard to absorbent articles such as catamenial devices (e.g. sanitary napkins, pantiliners, tampons etc.), diapers, bandages, adult incontinence garments, and the like; good liquid uptake and liquid-holding capacity are obvious prerequisites for the function of such articles. However, when used, an article of this kind is constantly subjected to pressure imposed by the weight and the movements of the bearer, and thus it is important that the liquid-holding capacity is high enough to retain the absorbed liquid also under pressure. Furthermore, in order to give good comfort to the bearer, the article should provide a feeling of dryness, meaning that any rewetting from the article to the skin of the bearer should by avoided, raising the requirements with regard to liquid-holding capacity even higher. Conventionally, certain polymeric materials forming hydrogels in contact with water, known as "superabsorbents", have been utilised to enhance the sorptive capacity of such articles; however, although this capacity of the article is enhanced, as the liquid is bound to superabsorbent particles, the sorptive capacity of the cellulosic fibres making up the fluff itself is not in fact enhanced by the use of such superabsorbents.

The problem to be solved by the present invention is thus to provide a method for production of fluff having improved sorptive capacity.

That problem is solved by the method defined by the appended claims.

The reason why hydrophobic substances of the stated kind impose improved sorption characteristics has so far not been clearly established.

In the present method a sheet of cellulosic fibres is shredded into fluff; in this context a "sheet" means a sheet or a web. The formation of the sheet, below referred to as "the pulp sheet", may follow either one of the paths for such formation known in the art, e.g. the wet method analogous to conventional paper making, or the flash drying method, both methods described in Pulp and Paper Manufacture, Vol. I, page 753-757, Joint Executive Committee of Vocational Education Committees of the Paper Industry, 1969.

To "treat" the fibres with a hydrophobic substance means that particles of the substance are brought into the proximity of, or in contact with the fibres; preferably, the particles are kept in the proximity of, or in contact with, the fibres for at least about 1 minute, suitably at least about 30 seconds, and particularly at least about 5 seconds. The treatment may be carried out at any point within the process starting with the separation of the fibres from each other and ending with dry shredding of the pulp sheet. Preferably, however, the treatment is carried out when the dry matter content of the fibres is at least about 25%, suitably at least about 35%, and most preferably at least about 50%.

The specific surface area of the hydrophobic substance particles, determined according to standard method DIN 66131 modified as described below, is at least about 50 m²/g, preferably at least about 100 m²/g, and most preferably at least about 1000 m²/g.

The hydrophobic substance may be any substance that is substantially insoluble in water, preferably having a solubility not higher than about 1 g/100 g of water, suitably not higher than about 0.1 g/100 g of water, as long as it has a specific surface area as indicated above. Exemplary of useful hydrophobic substances are activated carbon, hydrophobic zeolites, and polytetrafluoroethylene (i.e. Teflon). The substances are preferably porous. In a particularly preferred embodiment the hydrophobic substance is activated carbon or a zeolite having a hydrophobicity of below about 0.99 percent, preferably below about 0.90 percent, and suitably below about 0.70 percent by weight residual butanol as determined by the Residual Butanol Test described below. Especially preferred zeolites are those having a molar relation SiO₂/Al₂O₃ of at least 5.

Although the hydrophobic substance may be brought to the fibres in a dry as well as a wet state, it is preferred that the substance is comprised in an aqueous mixture, usually as a dispersion or a slurry. The mixture is suitably applied on the formed pulp sheet, conveniently by spraying. The amount of substance added is suitably about 0.1 - 10 kg/metric ton dry pulp, preferably about 0.5 - 5 kg/ton. Optionally the treatment may be carried out when the fibres are suspended in an aqueous solution, e.g. in the stock of the pulp production process prior to forming the sheet; the amount of substance in the stock is suitably about 0.1 - 10 kg/ton dry pulp, preferably about 0.5 - 5 kg/ton.

The treatment may be carried out in the presence of a retention agent to ensure that a sufficient amount of the substance particles are kept in contact with, or in the proximity of the fibres long enough to give the desired effect. Exemplary of preferred retention agents are polysaccharides, such as starch, cellulose derivatives, xanthan gum and guar gum, and synthetically produced homopolymers, such as polyacryl amide (PAM), polyamide amine (PAA), polydiallyl dimethyl ammonium chloride (polyDADMAC), polyethylene imine (PEI) and polyethylene oxide (PEO), or copolymers thereof.

Although the substance may remain in the fluff after shredding, this is not considered to be essential to the invention. In fact, even if less than about 65%, say less than 30%, or even as little as about 1% of the substance used in the treatment is present in the fluff after shredding, the sorption effect is quite noticeably enhanced when compared to fluff produced from untreated pulp. In some instances it may however be advantageous to let a substantial amount of substance remain in the fluff, for instance when the substance is a hydrophobic zeolite providing such odour eliminating characteristics to absorbent articles, e.g. diapers and catamenial devices, as disclosed in US-A-4,826,497 and WO-A-91/11977; it should, however, be noted that in those documents the zeolite is either used in positions separated from the fluff, or mixed with the fluff after shredding.

The present invention also relates to fluff obtainable by the present method. As is evident from the below examples such fluff shows surprisingly good sorption characteristics when compared to fluff mixed with zeolite or activated carbon according to prior art, i.e. after shredding. The cause of this enhanced effect is unknown, but is obviously due to some characteristic of the fluff imposed by the specific method of application.

The invention also relates to the use of a hydrophobic substance of the present kind for treatment of cellulosic fibres to improve the sorptive capacity of absorbent materials comprising the fibres, such as for instance fluff or hygienic paper, e.g. soft tissue. It furthermore relates to a process for the production of absorbent material, including for instance dry-formed nonwoven or tissue, that comprises cellulosic fibres treated as stated above with a hydrophobic substance prior to fluffing; it also relates to absorbent articles, such as sanitary napkins, pantiliners, tampons, diaper bandages, adult incontinence garments, and the like, in which fluff obtainable by the present method is used.

The fibres making up the pulp sheet are usually obtained by disintegrating wood, conventionally in the form of chips, into fibres or bundles of fibres; in the present context the concept of "bundles of fibres" is regarded to be equivalent to the concept of "fibres". The separated fibres may be obtained by means of any pulp-making method known to a skilled person, e.g. by a method for production of mechanical pulp (MP), stone groundwood pulp (SGW), pressure groundwood pulp (PGW), refiner mechanical pulp (RMP), thermomechanical pulp (TMP), chemi-mechanical pulp (CMP), or chemi-thermomechanical pulp (CTMP), although the preferred pulps are chemical pulps such as, for instance, sulphate and sulphite pulps. However, the cellulosic fibres may also advantageously be cotton fibres. Another plausible source of fibres is recycled fibres from wastepaper.

The present invention is illustrated in more detail below by means of examples. Unless otherwise stated the parts and percentages below are given by weight. In the examples the substances according to table I were used; substances A to N are zeolites and substance O is an activated carbon.

**Table I**

| Substance | hydrophobicity, % | molar relation SiO₂/Al₂O₃ | type |
|---|---|---|---|
| A | 0.03 | 900 | ZSM-5 |
| B | 0.14 | 35 | ZSM-5 |
| C | 0.15 | 35 | ZSM-5 |
| D | 0.24 | 29 | Y |
| E | 0.27 | 25 | Y |
| F | 0.28 | 29 | Y |
| G | 0.30 | 5.1 | Y |
| H | 0.46 | 12 | Y |
| I | 0.81 | 5.2 | Y |
| J | 0.81 | 5.2 | Y |
| K | 0.83 | 5.5 | Y |
| L | 0.99 | 2.6 | X |
| M | 0.99 | 2 | A |
| N | 0.99 | 2 | A |
| O | | | activated carbon |

The hydrophobicities of the zeolites indicated in Table I are determined by a so-called Residual Butanol Test, described in GB-A-2,014,970. In this test, the zeolite is activated by heating in air for 16 h at 300°C. Then, 10 parts by weight of the thus-activated zeolite are mixed with a solution consisting of 1 part by weight of I-butanol and 100 parts by weight of water. The resulting slurry is slowly agitated for 16 h at 25°C. Finally, the residual content of I-butanol in the solution is determined and indicated in per cent by weight. Thus, a low value indicates a high degree of hydrophobicity.

Activated carbon is the collective name for a group of porous carbons manufactured either by treatment of carbon with gases, or by carbonisation of carbonaceous materials with simultaneous activation by chemical treatment; a more detailed description of activated carbons is given in "Ullmann's encyclopedia of industrial chemistry" (Vol. A 5, page 124 and onwards), 1986.

The specific surface areas were determined by a method based on DIN 66131 (July 1993), the so-called BET method, in which the areas were determined by using one point of the adsorption isotherm at a relative pressure p/p₀ of 0.03, p being the pressure of the gas adsorbed in that method and p₀ being the saturation vapour pressure for the same gas. For activated carbon the specific surface area was about 1000 m2/g, for zeolite of type ZSM-5 and type A it was about 500 m2/g, whereas zeolite X and Y each showed a specific surface area of about 800 m2/g.

In the Examples below, the fluff obtained was tested with respect to rewetting, and in some Examples also with respect to the uptake rate. The test method for determination of the uptake rate was SCAN-C 33:80, in which fluff samples of 3 g having a diameter of 50 mm are positioned vertically and loaded with a weight of 500 g on top. The sample is allowed to absorb water from below, and the time taken until water penetrates through the upper surface of the sample is determined automatically by means of an electronic detector. The shorter time required to penetrate the upper surface, the higher is the uptake rate of the fluff. In the test method for determination of rewetting or liquid holding capacity a fluff sample of 3 g having a diameter of 50 mm is positioned vertically and loaded with a weight of 1 kg on top for 30 s, and is then unloaded. 10 ml of water is applied to the sample under a time period of 10 s and the liquid is allowed to drain the sample for 30 s, whereafter the sample is loaded with a weight of 1 kg for 4 minutes. 15 sheets, 8 x 8 cm, of filter paper are placed on top of the sample, and the combined sample and sheets are loaded with a weight of 5 kg for one additional minute, after which the 15 sheets are weighed. The increase of weight of the sheets is due to rewetting. Thus, a low weight increment indicates low rewetting.

Examples 1-14: 10 g sulphate pulp was slushed for 10 minutes in 500 ml water in a laboratory pulper. The thus obtained stock was dewatered through the screen cloth of a wire mould, producing pulp sheets having diameters of 210 mm. 15 g of aqueous solutions containing 0.2 % hydrophobic substance were sprayed onto the sheets in all examples except for Example 1, which is a comparison example. The amount of hydrophobic substance added to each sheet corresponded to 3 kg substance/ton of dry pulp. The sheets were dried at 60°C for 120 minutes and then dry shredded into fluff in a hammer mill. The fluff was formed into three samples, each of 3 g. The fluff samples thus obtained were tested, at ambient conditions of about 23°C and 50% RH, with regard to rewetting as is set forth in Table II below.

**Table II**

| Sulphate Pulp, 3 kg hydrophobic substance/metric ton pulp | | |
|---|---|---|
| | | substance added by spraying |
| substance | Ex | rewetting, g |
| Ref | 1 | 5.0 |
| A | 2 | 4.5 |
| B | 3 | 4.2 |
| C | 4 | 4.4 |
| D | 5 | 4.0 |
| F | 6 | 3.2 |
| G | 7 | 3.8 |
| H | 8 | 4.1 |
| I | 9 | 4.2 |
| J | 10 | 4.7 |
| L | 11 | 4.5 |
| M | 12 | 4.1 |
| N | 13 | 4.4 |
| O | 14 | 4.4 |

The fluff produced according to the present method evidently has a better liquid retaining capacity than untreated fluff.

Examples 15-21: The procedure used in Examples 1-14 was repeated, except that the hydrophobic substance was added during slushing instead of being sprayed. Here Example 15 is the comparison example, i.e. without any hydrophobic substance added. The fluff samples obtained were tested with regard to rewetting as is set forth in Table III below.

**Table III**

| Sulphate Pulp, 3 kg hydrophobic substance/ton pulp | | |
|---|---|---|
| | substance added by spraying | |
| substance | Ex | rewetting, g |
| Ref | 15 | 5.0 |
| B | 16 | 4.8 |
| C | 17 | 4.5 |
| D | 18 | 4.5 |
| F | 19 | 4.6 |
| G | 20 | 4.2 |
| I | 21 | 4.7 |

The fluff produced according to the present method evidently has a better liquid retaining capacity than untreated fluff.

Examples 22-26: The procedure outlined with regard to Examples 1-14 was repeated, although applied on sulphite pulp. Example 22 is a comparison example with no hydrophobic substance added. The results are set forth in Table IV below.

**Table IV**

| Sulphite Pulp, 3 kg hydrophobic substance/ton pulp | | |
|---|---|---|
| | substance added by spraying | |
| substance | Ex | rewetting, g |
| Ref | 22 | 5.15 |
| C | 23 | 4.37 |
| F | 24 | 4.22 |
| L | 25 | 4.84 |
| N | 26 | 4.55 |

The fluff produced according to the present method evidently has a better liquid retaining capacity than untreated fluff.

Examples 27-29: The procedure outlined with regard to Examples 1-14 was repeated, although applied on chemi-thermomechanical pulp (CTMP); uptake rates were also determined. Example 27 is a comparison example with no hydrophobic substance added. The results are set forth in Table V below.

**Table V**

| CTMP, 3 kg hydrophobic substance/ton pulp | | | |
|---|---|---|---|
| | substance added by spraying | | |
| substance | Ex | uptake rate, s | rewetting, g |
| Ref | 27 | 12 | 5.00 |
| C | 28 | 11 | 4.32 |
| F | 29 | 8.2 | 4.41 |

The fluff produced according to the present method evidently has a faster uptake rate and a better liquid retaining capacity than untreated fluff.

Examples 30-32: The procedure outlined with regard to Examples 1-14 was repeated, although applied on cotton; uptake rates were also determined. Example 30 is a comparison example with no hydrophobic substance added. The results are set forth in Table VI below.

**Table VI**

| Cotton, 3 kg hydrophobic substance/ton pulp | | | |
|---|---|---|---|
| | substance added by spraying | | |
| substance | Ex | uptake rate, s | rewetting, g |
| Ref | 30 | 5.1 | 6.54 |
| C | 31 | 4.6 | 6.24 |
| F | 32 | 4.4 | 6.00 |

The fluff produced according to the present method evidently has a faster uptake rate and a better liquid retaining capacity than untreated fluff.

Examples 33-38: The procedure outlined with regard to Examples 1-14 was repeated, although in the test method for determination of rewetting an aqueous solution the properties relevant for the test of which were similar to those of blood was used. This solution contained 10 g/l of NaCI, 80 g/l of glycerol, 4 g/l of NaHCO₃; the viscosity of the solution was adjusted to about 12 cP by adding carboxymethyl cellulose (CMC), and the surface tension was adjusted to about 50 mN/m by adding a nonionic surfactant. Example 33 is a comparison example with no hydrophobic substance added. The results are set forth in Table VII below.

**Table VII**

| Sulphate Pulp, 3 kg hydrophobic substance/ton pulp | | |
|---|---|---|
| | substance added by spraying | |
| substance | Ex | rewetting, g |
| Ref | 33 | 4.50 |
| E | 34 | 3.81 |
| F | 35 | 3.60 |
| H | 36 | 3.96 |
| K | 38 | 4.26 |

Thus, also with regard to blood the fluff produced according to the present method has a better liquid retaining capacity than untreated fluff.

Example 39: substance F was added to sulphate pulp, directly to the stock or by spraying a pulp sheet. The treated pulp was then shredded into fluff, and the remaining content of substance F in the fluff was determined. Furthermore a comparative test, according to prior art, in which dry substance F was applied to dry-shredded sulphate pulp (i.e. fluff) was carried out. These three fluff samples were tested with regard to rewetting. The results are set forth in Table VIII below.

**Table VIII**

| Application method | Added amount of substance F, kg/t | Remaining amount of substance F in fluff, kg/t | Rewetting, g |
|---|---|---|---|
| Dry hydrophobic substance on dry fluff (prior art) | 0.6 | 0.6 | 4.56 |
| Addition to stock | 3 | 0.6 | 4.44 |
| Addition by spraying | 1 | 0.6 | 4.35 |

Although the three obtained fluff products contained the same amount of substance F, their properties were thus clearly different, as evidenced by the fact that the fluff produced according to the present method had significantly better rewetting characteristics.

## Claims

1. A method for production of fluff having improved sorptive capacity, in which a sheet of cellulosic fibres is shredded into fluff, **characterised** in that prior to shredding the sheet the fibres are treated in the presence of water with a hydrophobic substance having a specific surface area of at least about 50 m²/g.

2. A method according to claim 1, **characterised** in that prior to the treatment the fibres have an dry content of at least about 20%.

3. A method according to claim 1 or 2, **characterised** in that the substance is activated carbon or a zeolite having a hydrophobicity of below about 0.99 percent by weight residual butanol as determined by the Residual Butanol Test.

4. A method according to any one of claims 1-3, **characterised** in that the fibres are sprayed or showered with a mixture comprising the substance and water.

5. A method according to any of claims 1-4, **characterised** in that the fibres are suspended in an aqueous solution during the treatment, prior to forming the sheet.

6. Fluff obtainable by a method according to any preceding claim.

7. An absorbent article comprising fluff according to claim 6.

8. Use of a hydrophobic substance having a specific surface area of more than 50 m²/g for treatment of cellulosic fibres to improve the sorptive capacity of absorbant material comprising the fibres.

9. Use according to claim 8, **characterised** in that the hydrophobic substance is activated carbon or a zeolite having a hydrophobicity of below about 0.99 percent by weight residual butanol as determined by the Residual Butanol Test.

10. A process for the production of absorbent material comprising cellulosic fibres obtained by shredding a sheet of such fibres, which structure has improved sorptive capacity, **characterised** in that the cellulose fibres, prior to shredding the sheet, have been treated in the presence of water with a hydrophobic substance having a specific surface area of at least 50 m²/g.

11. Absorbant material obtainable from a process according to claim 10.

12. Absorbant material according to claim 11, **characterised** in that the material is dry-formed nonwoven or tissue.

13. Absorbant material according to claim 11, **characterised** in that the material is fluff.

## Patentansprüche

1. Verfahren zur Herstellung von Fluff mit verbesserter Sorptionskapazität, wobei eine Bahn aus Cellulosefasern zu Fluff zerkleinert wird, dadurch gekennzeichnet, dass die Fasern vor dem Zerkleinern der Bahn in Gegenwart von Wasser mit einer hydrophoben Substanz behandelt werden, die eine spezifische Oberfläche von mindestens etwa 50 m²/g hat.

2. Verfahren nach Anspruch 1, dadurch gekennzeichnet, dass die Fasern vor der Behandlung einen Trockengehalt von mindestens etwa 20% aufweisen.

3. Verfahren nach Anspruch 1 oder 2, dadurch gekennzeichnet, dass die Substanz Aktivkohle oder ein Zeolith mit einer durch den Rest-Butanoltest bestimmten Hydrophobie unter etwa 0,99 Gew.% Rest-Butanol ist.

4. Verfahren nach einem der Ansprüche 1 bis 3, dadurch gekennzeichnet, dass die Fasern mit einem die Substanz und Wasser umfassenden Gemisch besprüht oder berieselt werden.

5. Verfahren nach einem der Ansprüche 1 bis 4, dadurch gekennzeichnet, dass die Fasern während der Behandlung und vor der Herstellung der Bahn in einer wässrigen Lösung suspendiert werden.

6. Fluff, erhältlich durch ein Verfahren nach einem vorhergehenden Anspruch.

7. Absorbierender Gegenstand, umfassend Fluff nach Anspruch 6.

8. Verwendung einer hydrophoben Substanz mit einer spezifischen Oberfläche von mehr als 50 m²/g zur Behandlung von Cellulosefasern zur Verbesserung der Sorptionskapazität des die Fasern umfassenden absorbierenden Materials.

9. Verwendung nach Anspruch 8, dadurch gekennzeichnet, dass die hydrophobe Substanz Aktivkohle oder ein Zeolith mit einer durch den Rest-Butanoltest bestimmten Hydrophobie unter etwa 0,99 Gew.% Rest-Butanol ist.

10. Verfahren zur Herstellung von absorbierendem Material, umfassend Cellulosefasern, die durch Zerkleinern einer Bahn solcher Fasern erhalten werden, wobei die Struktur verbesserte Sorptionskapazität hat, dadurch gekennzeichnet, dass die Cellulosefasern vor dem Zerkleinern der Bahn in Gegenwart von Wasser mit einer hydrophoben Substanz mit einer spezifischen Oberfläche von mindestens 50 m²/g behandelt worden sind.

11. Absorbierendes Material, erhältlich durch ein Verfahren nach Anspruch 10.

12. Absorbierendes Material nach Anspruch 11, dadurch gekennzeichnet, dass das Material ein trocken hergestelltes Faservlies oder Gewebe ist.

13. Absorbierendes Material nach Anspruch 11, dadurch gekennzeichnet, dass das Material Fluff ist.

## Revendications

1. Procédé de production de duvet de peluchage ayant un pouvoir de sorption accru, dans lequel une feuille de fibres cellulosiques est déchiquetée en duvet de peluchage, **caractérisé** en ce que, avant le déchiquetage de la feuille, les fibres sont traitées en présence d'eau par une substance hydrophobe ayant une surface spécifique d'au moins environ 50 m²/g.

2. Procédé selon la revendication 1, **caractérisé** en ce que, avant le traitement, les fibres ont une teneur en matière sèche d'au moins environ 20 %.

3. Procédé selon la revendication 1 ou 2, **caractérisé** en ce que la substance est du charbon actif ou une zéolithe ayant un degré d'hydrophobie, déterminé par le test au butanol résiduaire, correspondant à moins d'environ 0,99 % en poids de butanol résiduaire.

4. Procédé selon l'une quelconque des revendications 1 à 3, **caractérisé** en ce que l'on pulvérise ou fait pleuvoir sur les fibres un mélange comprenant la substance et de l'eau.

5. Procédé selon l'une quelconque des revendications 1 à 4, **caractérisé** en ce que les fibres sont mises en suspension dans une solution aqueuse pendant le traitement, avant la formation de la feuille.

6. Duvet de peluchage que l'on peut obtenir par un procédé selon l'une quelconque des revendications précédentes.

7. Article absorbant, renfermant du duvet de peluchage selon la revendication 6.

8. Utilisation d'une substance hydrophobe ayant une surface spécifique supérieure à 50 m²/g pour le traitement de fibres cellulosiques, afin d'augmenter le pouvoir de sorption d'une matière absorbante contenant les fibres.

9. Utilisation selon la revendication 8, **caractérisée** en ce que la substance hydrophobe est du charbon actif ou une zéolithe ayant un degré d'hydrophobie, déterminé par le test au butanol résiduaire, correspondant à moins d'environ 0,99 % en poids de butanol résiduaire.

10. Procédé de production d'une matière absorbante comprenant des fibres cellulosiques obtenues par déchiquetage d'une feuille desdites fibres, ladite structure ayant un pouvoir de sorption accru, **caractérisé** en ce que, avant le déchiquetage de la feuille, les fibres de cellulose ont été-traitées en présence d'eau par une substance hydrophobe ayant une surface spécifique d'au moins 50 m²/g.

11. Matière absorbante que l'on peut obtenir par le procédé selon la revendication 10.

12. Matière absorbante selon la revendication 11, **caractérisée** en ce qu'elle est un tissu ou un non-tissé formé à sec.

13. Matière absorbante selon la revendication 11, **caractérisée** en ce qu'elle est du duvet de peluchage.
